# EUROPEAN PATENT SPECIFICATION

(11) **EP 1 754 061 B1**
(45) Date of publication and mention of the grant of the patent: **22.10.2008**
(21) Application number: 05754413.2
(22) Date of filing: 06.06.2005
(51) Int. Cl.: G01N 33/574, G01N 33/50

(54) **BRCA1 MARKERS**
BRCA1-MARKER
MARQUEURS BRCA1

(30) Priority: 05.06.2004 GB 0412620
(43) Date of publication of application: 21.02.2007
(73) Proprietor: The Queens University of Belfast, Belfast BT7 1NN (GB)
(72) Inventor: HARKIN, Paul Department of Oncology, Belfast BT9 7AB (GB)
(74) Representative: Thom, Russell
(86) International application number: PCT/GB2005/002227
(87) International publication number: WO 2005/121786

(56) References cited:
- WO-A-00/26668
- WO-A-03/057159
- US-A1- 2003 165 954
- OLOUMI A ET AL: "Identification of genes differentially expressed in V79 cells grown as multicell spheroids" INTERNATIONAL JOURNAL OF RADIATION BIOLOGY, vol. 78, no. 6, June 2002 (2002-06), pages 483-492, XP008053115 ISSN: 0955-3002
- RICE JUDD C ET AL: "Methylation of the BRCA1 promoter is associated with decreased BRCA1 mRNA levels in clinical breast cancer specimens" CARCINOGENESIS (OXFORD), vol. 21, no. 9, September 2000 (2000-09), pages 1761-1765, XP002346860 ISSN: 0143-3334
- DATABASE BIOSIS [Online] BIOSCIENCES INFORMATION SERVICE, PHILADELPHIA, PA, US; 2005, GORSKI JULIA ET AL: "BRCA1 and c-Myc associate to transcriptionally repress psoriasin" XP002346862 Database accession no. PREV200510146285 & CELLULAR ONCOLOGY, vol. 27, no. 2, 2005, page 149, MEETING OF THE INTERNATIONAL-SOCIETY-FOR-CELLULAR-ONCOLOG Y (ISCO 2005); BELFAST, NORTH IRELAND; APRIL 05 -08, 2005 ISSN: 1570-5870

## Description

### TECHNICAL FIELD

The invention relates to a method of determining the presence of a non-functional BRCA1 gene in a biological sample or reduced expression of a functional BRCA1 gene.

### BACKGROUND ART

The BRCA1 tumour suppressor gene was identified by positional cloning as one of the genes conferring genetic predisposition to breast and ovarian cancer. BRCA1 mutation carriers have recently been reported to have an 82% risk of developing breast cancer and a 54% risk of developing ovarian cancer by the age of 80. Although the exact function of BRCA1 remains to be defined, roles in DNA damage detection, DNA damage repair, in particular repair of double strand breaks, cell cycle checkpoint control, in particular S-phase and G2 arrest following ionizing irradiation, transcriptional regulation and ubiquitination have been inferred.

As BRCA1 has been identified as one of the genes conferring genetic predisposition to breast and ovarian cancer, it is desirable to provide a test which screens an individual for the presence of mutant forms of BRCA1. However, as many different mutations have been identified within the BRCA1 gene, it is difficult to design a rapid and effective means of assaying for each and every possible mutation. To overcome this problem of intragenic heterogeneity, it is necessary to sequence the whole gene from patients and interrogate the obtained sequence for mutations. While such a whole gene sequencing approach provides an accurate diagnostic test for BRCA1 mutation carriers, the test itself is very expensive and time consuming. Rice et al. (Carcinogenesis, vol. 21, no. 9, pp 1761-1765) disclose a method for determining the status of BRCA1.

It is an object of the invention to overcome at least some of the above problems.

### Summary of the Invention

The present inventor has surprisingly determined that specific members of the S100 family of proteins are potently repressed by BRCA1 while other members are upregulated by BRCA1 and therefore may act as surrogate markers of BRCA1 deficiency.

Accordingly, in the present invention, there is provided a method of predicting the presence of a non-functional BRCA1 gene in a test biological sample comprising the steps of:
assaying the test sample to determine an expression profile of at least one member of the S100 family of genes selected from the group comprising S100A7, S100A8, S100A9, S100A10 or S100A2, and
comparing the expression profile of at least one member with the expression profile of said member in a control biological sample,
wherein an increase in the expression of any one of S100A7, S100A8, S1009A9, or S100A10 or a decrease in expression of S100A2 in the test sample being assayed is indicative of non-functional BRCA1.

In addition to expression of non-functional BRCA1, BRCA1 deficiency can be also be due to the reduced expression of functional BRCA1 due to, for example, epigenetic modification of BRCA1. For the purposes of the present invention, the reduced expression of functional BRCA1 is considered to be encompassed by "non-functional BRCA1".

Typically, the test biological sample will be a sample of tissue. Any suitable tissue may be used. However, it is also possible to assay biological fluids such as blood for expression of the gene of interest. This is particularly the case given that the S100 family of proteins are members of the secretome of any given cell.

The control biological sample is preferably a sample of similar cell type or biological fluid to the test sample, but in which BRCA1 is known to be functional and in which BRCA1 is expressed at normal (non-disease) levels.

Preferably breast or ovarian suspected tumour tissue is used as the test sample and normal breast or ovarian tissue is used as a non-test or control sample.

The presence of non-functional BRCA1 has been determined to be influential in determining which suitable chemotherapeutic treatment agent should be provided to an individual.

Thus the present invention can be used to predict sensitivity to agents which demonstrate enhanced effects in neoplasms characterised by non-functional BRCA1.

Thus, optionally, the method of the first aspect of the invention further comprises the step of determining a chemotherapeutic agent which would be suitable for treatment of individuals from which the test sample is derived.

Typically, when it is determined that the expression profile of a member of the S100 family of genes selected from the group S100A7, S100A8, S100A9 or S100A10 is increased and / or the expression of S100A2 is decreased from the expression levels profiled in the non-test (control) sample, a DNA damaging agent is chosen as a chemotherapeutic agent.

Any suitable DNA damaging chemotherapeutic agent may be used. The agents may include: platinum agents e.g. cisplatin, etoposide, doxorubicin, bleomycin and cyclophosphamide. Other suitable DNA damaging agents will be known to those skilled in the art.

In contrast, when the expression profile of a member of the S100 family of genes selected from the group comprising S100A7, S100A8, S100A9, S100A10 or S100A2 is normal, i.e. similar to that of the non test (control) and thus is indicative of functional BRCA1, an antimicrotubule chemotherapeutic agent may be chosen.

Suitably, the antimicrobule agent is selected from the group comprising: taxol, paclitaxel, taxatere, docetaxel and vinorelbine.

Treatment protocols for treating an individual with a chemotherapeutic antimicrotubule agent, or with a DNA damaging chemotherapeutic agent, will be well known to those skilled in the art of chemotherapy and will not be discussed further.

As discussed in the Examples, the inventor has surprisingly shown that the expression of the S100 protein psoriasin, markedly enhances the sensitivity of cancer cells to DNA damaging agents.

Also disclosed is a method of predicting the sensitivity of a test sample of cancer cells to treatment with a DNA damaging agent comprising the steps of
determining the expression profile of at least one member of the S100 family of genes selected from the group comprising S100A7, S100A8, S100A9, S100A10 or S100A2, and
comparing the expression profile of at least one member with the expression profile of said member in a control biological sample,
wherein an increase in the expression of any one of S100A7, S100A8 or S1009A9, S100A10 or a decrease in expression of S100A2 in the test sample being assayed is indicative that the cells are sensitive to DNA damaging agents.

Mutations in the BRCA1 tumour suppressor gene has been indicated to confer a genetic predisposition to particular types of cancer.

Also disclosed is an in-vitro assay method of detecting a genetic predisposition to cancer, comprising the steps of;
assaying a test biological sample to determine the expression profile of a member of the S100 family of genes selected from the group comprising S100A7, S100A8, S100A9, S100A10 or S100A2, and
comparing the expression profile of at least one member with an expression profile of said member obtained from a sample(s) from an individual(s) who does not have a genetic predisposition to cancer,
wherein an increase in the expression of any one of S100A7, S100A8, S1009A9, or S100A10 or a decreased expression of S100A2 in the test sample being assayed is indicative that the individual from whom the sample has been provided has a greater likelihood of a genetic predisposition to cancer.

The method may be used to determine a predisposition to any type of cancer in which non-function of BRCA1 has been implicated.

Suitably, the cancer is selected from the group comprising: breast cancer; ovarian cancer; early onset breast cancer; early onset ovarian cancer; sporadic breast cancer; sporadic ovarian cancer; and invasive and non-invasive breast and ovarian cancer

Preferably, the methods of the invention determine if the expression level of S100A7 in the test sample is increased in comparison to the expression level of S100A7 in a non-test sample.

In embodiments of the methods of the invention, the expression profiles of two S100 genes selected from the group comprising S100A7, S100A8, S100A9, S100A10 or S100A2 are determined by the assay of the biological sample and compared with the expression profiles of said members in a non-test biological sample.

For example the following combinations of S100 members may be assayed and compared with the respective members in a non-test sample;
S100A7 + S100A8,
S100A7 + S100A9,
S100A7 + S100A2,
S100A7 + S100A10
S100A8 + S100A9,
S100A8 + S100A10
S100A9 + S100A10
S100A8 + S100A2, or
S100A9 + S100A2
S100A10 + S100A2

In particular embodiments of the methods of the invention, the expression profiles of three S100 genes selected from the group comprising S100A7, S100A8, S100A9, S100A10 or S100A2 are determined by the assay of the test biological sample and compared with the expression profiles of said members in a non-test sample.

For example the following combinations of S100 members may be assayed and compared with the respective members in a non-test sample;
S100A7 + S100A8 + S100A9,
S100A7 + S100A8 + S100A10
S100A7 + S100A9 + S100A10
S100A7 + S100A8 + S100A2,
S100A7 + S100A9 + S100A2,
S100A7+ S100A10 + S100A2
S100A8 + S100A9 + S100A2
S100A8 + S100A9 + S100A10, or
S100A9 + S100A10 + S100A2.

In a further embodiment of the methods of the invention the expression profiles of four of S100A7, S100A8, S100A9, S100A10 or S100A2 are determined by an assay of the test biological sample and compared with the expression profiles of said members in a non-test sample.

In a further embodiment of the methods of the invention the expression profiles of each of S100A7, S100A8, S100A9, S100A10 and S100A2 are determined by an assay of the test sample and compared with the expression profiles of said members in a non-test sample.

In a preferred embodiment, the assay is designed to detect the expression of a protein product of at least one member of the S100 family of genes selected from the group consisting of S100A7, S100A8, S100A9, S100A10 or S100A2.

A sample may be tested for the presence of at least one member of S100A7, S100A8, S100A9, S100A10 or S100A2 protein using a specific binding member such as an antibody (or mixture of antibodies), specific for said at least one member.

In such cases the sample may be tested by providing a specific binding member such as an antibody to the sample under specific conditions for specific binding and for instance using a suitable reporter system to indicate whether binding of the specific member to at least one member of the S100 family of genes selected from the group comprising of S100A7, S100A8, S100A9, S100A10 or S100A2 has occurred.

Any suitable antibodies for S100A7, S100A8, S100A9, S100A10 or S100A2 may be used. Such antibodies may be available commercially, sources of which will be known to those skilled in the art. For example, S100A7 (Psoriasin) antibody is available from Imgenex; S100A8 and / or S100A9 antibodies are available from Bachem.

In a particularly preferred embodiment of the invention, the assay will employ a suitably designed ELISA (Enzyme Linked Immunosorbent Assay).

Examples of suitable reporting systems, which may for example be used in a suitably designed ELISA, to indicate whether binding of the specific member to at least one member of the S100 family of genes selected from the group comprising of S100A7, S100A8, S100A9, S100A10 or S100A2 has occurred may include horseradish peroxidase, malate dehydrogenase, staphylococcal nuclease, delta-5-steroid isomerase, yeast alcohol dehydrogenase, alpha-glycerophosphate dehydrogenase, triose phosphate isomerase, alkaline phosphatase, asparaginase, glucose oxidase, beta-galactosidase, ribonuclease, urease, catalase, glucose-6-phosphate dehydrogenase, glucoamylase, and acetylcholine esterase.

Suitably, for increased sensitivity in the ELISA system, the reporting system may use biotinylated antibody reacting with avidin-peroxidase conjugates. Other methods of detecting biotinylation may further be used. Such methods will be apparent to the man skilled in the art and include detection systems involving streptavidin and systems including peroxidase and alkaline phosphatase detecting systems.

The amount of at least one specific S100 protein may also be determined by labelling an antibody which specifically binds to a member of the S100 protein family with a radioactive isotope. The presence of the radioactive isotope would then be determined by such means as the use of a gamma counter or a scintillation counter. Isotopes which are particularly useful are 3H, 125I, 1231, 32P, 35S, 14C, 51Cr, 36C1, 57Co, 58Co, 59we, 75Se, 111In 99mTc, 67Ga, and Y.

Determination of the amount of at least one specific S100 protein is also possible by labelling an antibody which specifically binds to a member of S100 protein family with a fluorescent compound. When the fluorescently labelled molecule is exposed to light of the proper wavelength, its presence can then be detected due to fluorescence of the dye. Among the most important fluorescent labelling compounds are fluorescein isothiocyanate, rhodamine, phycoerythrin, phycocyanin, allophycocyanin, o-phthaldehyde, and fluorescamine.

Fluorescence emitting metal atoms such as Eu (europium), and other lanthanides, can also be used. These can be attached to the desired molecule by means of metalchelating groups, such as DTPA or EDTA.

Another way in which the antibody can be detectably labelled is by coupling it to a chemiluminescent compound. The presence of the chemiluminescent-tagged immunoglobulin is then determined by detecting the presence of luminescence that arises during the course of a chemical reaction. Examples of particularly useful chemiluminescent labelling compounds are luminol, isoluminol, aromatic acridinium ester, imidazole, acridinium salt, and oxalate ester.

Likewise, a bioluminescent compound may also be used as a label. Bioluminescence is a special type of chemiluminescence which is found in biological systems and in which a catalytic protein increases the efficiency of the chemiluminescent reaction. The presence of a bioluminescent molecule would be determined by detecting the presence of luminescence. Important bioluminescent compounds for purposes of labelling are luciferin, luciferase, and aequorin.

The use of chemiluminescent substrates for HRP and alkaline phosphatase could also be used.

Other methods of detecting the presence of a particular protein such as, for example, PAGE, isoelectric focussing and western blotting may be used. Such methods will be well known to a person skilled in the art.

Rather that detecting the presence of the (protein) gene product, the expression of the gene in question may be evaluated by assaying for mRNA transcripts of the gene, or cDNA clones of the mRNA transcripts. Methods for detecting specific mRNA or cDNA molecules will be well known to those skilled in the art, for example RT-PCR, Northern Blot or microarray analysis.

In one preferred embodiment, real-time PCR (RT-PCR) is used to assay mRNA transcripts.

Several RT-PCR systems are commercially available, for example, but not limited to TaqMan® (Applied Biosystems, Foster City, CA, USA), Molecular Beacons, Scorpions® and SYBR® Green (Molecular Probes), are available for. All of these chemistries allow detection of PCR products via the generation of a fluorescent signal.

Also disclosed is a kit for predicting the presence of non-functional BRCA1, the reduced expression of functional BRCA1, or a genetic predisposition to cancer, said kit comprising means for assaying a sample for expression of at least one member of the S100 gene family selected from the group comprising S100A7, S100A8, S100A9, S100A10 or S100A2.

Regarding the kit for determining the presence of protein of members of the S100 family selected from the group comprising S100A7, S100A8, S100A9, S100A10 or S100A2, the kit may include an antibody able to bind at least one member selected from the group comprising S100A7, S100A8, S100A9, S100A10 or S100A2, and / or reagents for a reporting system as discussed above and / or means for providing the test sample, for example a fine needle for fine needle aspiration of a tissue sample or other tissue collection means.

Suitably, the kit will assay for a specific S100 gene product using a suitably designed ELISA assay

Regarding the kit for determining the presence the mRNA transcripts for members of the S100 family selected from the group comprising S100A7, S100A8, S100A9, S100A10 or S100A2, the kit may include reagents for use in PCR, such as polymerase, nucleosides, buffer solutions etc, means for providing the test sample, for example a fine needle for fine needle aspiration of a tissue sample or other tissue collection means.

The kit may suitably be provided with instructions for use of the kit.

Preferred features of each aspect of the invention are as for each of the other aspects *mutatis mutandis* unless the context demands otherwise.

### Definitions

The term "predicting the presence" as used herein should be taken to mean estimating the possibility of the gene being non-functional. In this regard, it should be understood that the invention does not always provide a concrete diagnosis of the presence of a non-functional gene, nor does it provide merely a vague indication that a non-functional gene may be present. Rather, the method of the invention provides a means of screening a population using a rapid and cost effective screening process with a view to isolating those members of the population who have an increased risk of carrying non-functional BRCA1 genes. Typically, those identified by the method of the invention will have a high risk of being BRCA1 mutation carriers.

The term "non-functional" as used herein refers to genes which carry mutations or which are silenced due to epigenetic inactivation. Inactivation may be partial or complete.

The term "expression profile" as used herein should be taken to include expression, over expression and reduced expression of the gene.

The term "method of determining a suitable chemotherapeutic agent for an individual" as used herein should be understood to mean a predictive estimation of a suitable chemotherapeutic agent.

The role played by BRCA1 in mediating the phenotypic response to a range of chemotherapeutic agents commonly used in cancer treatment has previously been evaluated. Here, a mechanism to explain how BRCA1 deficiency leads to sensitivity to DNA damaging agents such as etoposide is provided. Specifically, it is demonstrated herein that the S100 family of proteins including S100A7 (Psoriasin), S100A8, S100A9 and S100A10 are DNA damage inducible and potently repressed by BRCA1 suggesting that overexpression of these proteins represents a surrogate marker of BRCA1 deficiency.

In addition it is shown that psoriasin overexpression mediates sensitivity to etoposide and conversely the inhibition of endogenous psoriasin results in resistance to etoposide, suggesting that psoriasin expression levels may predict for response to DNA damage based chemotherapeutic agents. Finally it is demonstrated that the mechanistic basis of psoriasin induced sensitivity to etoposide is due to its ability to inhibit the expression of the cdk inhibitor p27.

The data therefore identifies specific members of the S100 family of proteins as physiologically important targets of BRCA1 and indicates that they function as predictive markers of chemotherapy response.

Embodiments of the present invention will now be described, by way of example only, with reference to the accompanying figures, in which:
Figure 1 shows microarray analysis comparing gene expression profiles between the HCC-BR116 and HCC-EV1 Cell Lines (a) Unsupervised hierarchical clustering demonstrating segregation of the HCC-BR116 and HCC-EV1 cell lines into two discrete groups, (b) The S100A family genes psoriasin (S100A7), S100A8 and S100A9 are expressed at lower levels in the HCC-BR116 cell line when compared to the HCC-EV1 cell line;

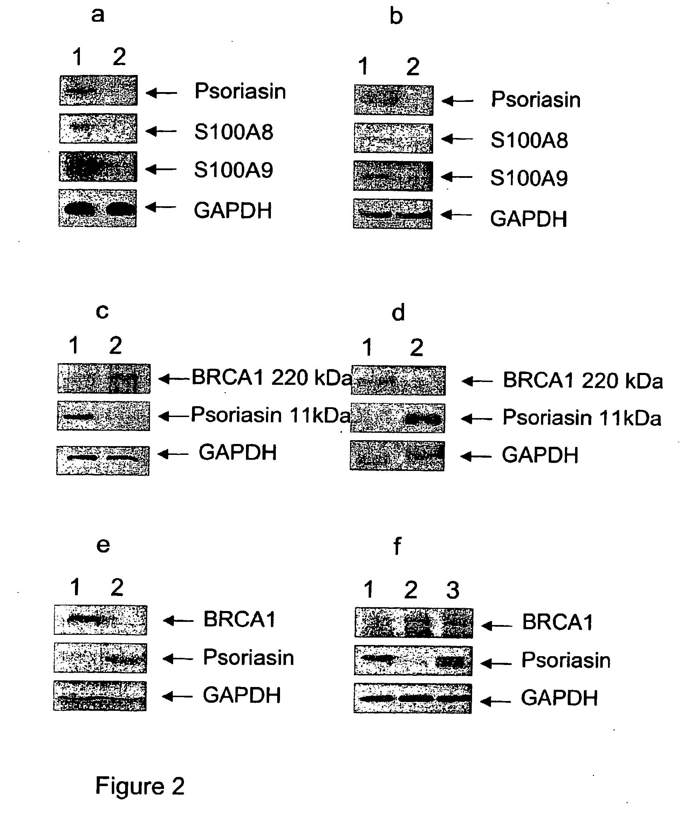

Figure 2 shows BRCA1 represses S10A7 Psoriasin, S100A8 and S100A9 at mRNA and Protein Level**(a)** Northern blot demonstrating repression of psoriasin, S100A8 and S100A9 mRNA in the HCC-BR116 cell line (lane 2) compared to the HCC-EV1 cell line (lane 1). Reprobing for GAPDH confirmed equal RNA loading, **(b)** Western blot demonstrating repression of psoriasin, S100A8 and S100A9 mRNA in the HCC-BR116 cell line (lane 2) compared to the HCC-EV1 cell line (lane 1). Reprobing for GAPDH confirmed equal RNA loading, **(c)** Western blot demonstrating repression of psoriasin 48 hours following transient transfection of the pRcCMV-BRCA1 vector into the HCC1937 cell line (lane 2) compared to a vector only transfected control (lane 1). Reprobing for GAPDH confirmed equal protein loading and a Western blot confirmed expression of exogenous BRCA1 (lane 2), **(d)** Western blot demonstrating expression of psoriasin in the AS4 cell line stably expressing a BRCA1 antisense construct (lane 2) compared to the vector only transfected BG1-neo cell line (lane 1). Repression of BRCA1 in the antisense transfected cells (lane 2) is demonstrated when compared to vector transfected controls (lane 1). Reprobing for GAPDH confirmed equal protein loading, **(e)** Western blot assay demonstrating expression of psoriasin 72 hours following transfection of a BRCA1 specific siRNA oligonucleotide into the T47D cell line (lane 2) compared to a scrambled oligonucleotide transfection control (lane 1). Reduced expression of BRCA1 in the SiRNA transfected cells (lane 2) compared to a scrambled control (lane 1) is demonstrated. Reprobing for GAPDH confirmed equal protein loading, **(f)** Western blot demonstrating that exogenous BRCA1 encoding the disease associated mutation E5243A fails to repress psoriasin in the HCC1937 cells (lane 3) compared to exogenous wild type BRCA1 (lane 2). HCC1937 cells were also transfected with an empty vector as a control (lane 1). Blotting for BRCA1 confirmed that the mutant BRCA1 (lane 3) was expressed at levels comparable to the wild type BRCA1 (lane 2). Reprobing for GAPDH confirmed equal protein loading;
Figure 3 confirms that BRCA1 is required for repression of psoriasin using multiple model systems;
Figure 4 demonstrates that BRCA1 forms a repressor complex with C-Myc on the psoriasin promoter;
Figure 5 demonstrates that psoriasin expression levels predict response to etoposide;
Figure 6 demonstrates that Psoriasin modulates sensitivity/resistance to etoposide through the modulation of p27 expression levels; and

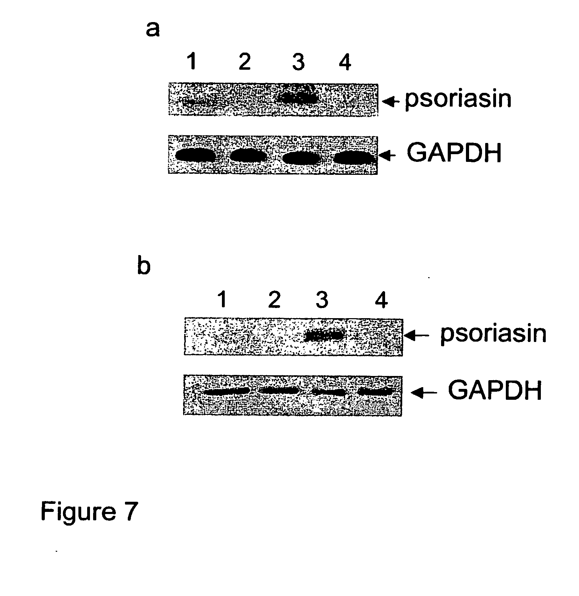

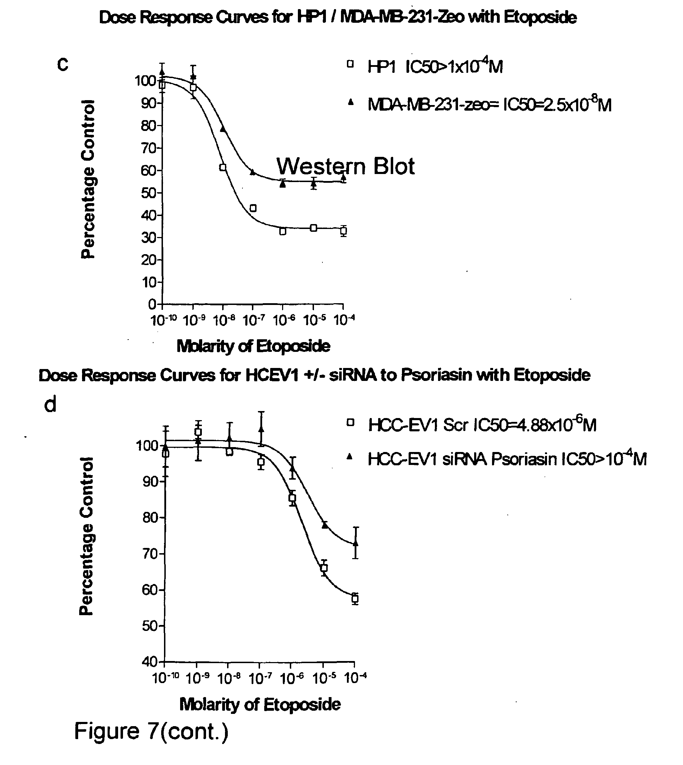

Figure 7 shows **(a)** A Northern blot evaluating the expression of psoriasin mRNA in the HCC-EV1 cell line (lanes 1 and 3) and the HCC-BR116 cell line (lanes 2 and 4). Cells were either untreated (lanes 1 and 2) or treated with 1µM etoposide for 24 hours. The blot was reprobed for GAPDH as a loading control, **(b)** A Western blot evaluating the expression of psoriasin protein in the HCC-EV1 cell line (lanes 1 and 3) and the HCC-BR116 cell line (lanes 2 and 4). Cells were either untreated (lanes 1 and 2) or treated with 1µM etoposide for 24 hours. The blot was reprobed for GAPDH as a loading control, **(c)** A 72 hour dose inhibition study investigating the effect of psoriasin expression on the response to etoposide in the HP1 and MDA-MB-231-zeo cell lines. The IC₅₀ values were calculated from the sigmoidal dose response curves. The results represent three independent experiments. (Symbols □ = HP1 cell line (psoriasin expression) ▲ = MDA-MB-231-zeo (empty vector control), **(d)** A 72 hour dose inhibition study investigating the effect of loss of psoriasin expression on the response to etoposide in the HCCEV1 cell line. The IC₅₀ values were calculated from the sigmoidal dose response curves. The results represent three independent experiments. (Symbols ▲ = siRNA to psoriasin □ =scrambled sequence).

### Experimental

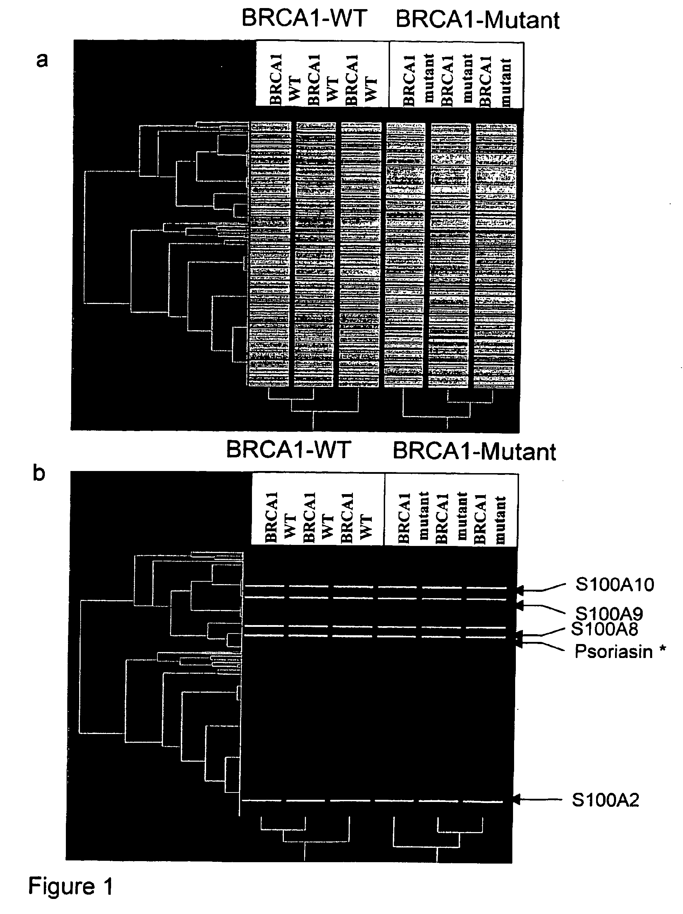

A microarray based expression profiling expression approach was used to compare gene expression profiles of cells containing functional or non-functional (mutant) BRCA1. The cell lines used were derived from the BRCA1-mutant HCC1937 cell line by stable expression of either wild-type exogenous BRCA1 (HCC-BR116) or an empty vector (HCC-EV1). Total RNA was extracted in triplicate from each cell line, biotinylated, and hybridised to the Affymetrix human genome U133 Plus 2.0 representing 47,000 known transcripts and expressed sequence tags. Analysis of the microarray data revealed 303 genes that exhibited BRCA1-dependent expression changes. Unsupervised hierarchical clustering of genes based on their expression profiles, and samples based on their expression signature was performed using Pearson correlation as the similarity metric resulting in 2-dimensional dendrogram. The clustering of samples or condition tree is shown vertically, and the clustering of genes or gene tree is shown horizontally. The samples diverged into two distinct clusters representing the HCC-BR116 and HCC-EV1 samples respectively. The gene clusters also diverged into two main groupings at the metanode of the tree with 189 genes induced by BRCA1 and 114 genes repressed (Figure 1a). Of particular interest were the S100A gene family members including psoriasin (S100A7), S100A8 and S100A9 that were etoposide inducible but markedly repressed by BRCA1. These are displayed as a subset of the original tree (Figure 1b). Northern and Western blot analysis confirmed that psoriasin, S100A8 and S100A9 were potently repressed by BRCA1 in agreement with the microarray data (*Figures 2a & b*).

To ensure that the observed BRCA1-mediated repression of psoriasin expression was not a clonal artefact of the HCC-EV1 and HCC-BR116 cell line models, the experiments were repeated transiently. The HCC1937 cell line was transiently transfected with either pRcCMV-BRCA1 or an empty pRcCMV vector and protein lysates were extracted after 48 hours. Transient expression of exogenous BRCA1 dramatically reduced psoriasin expression indicating that the observed effect in the HCC-BR116 cell line was not due to a clonal artefact (Figure 2c). To further emphasise the significance of this effect this analysis was extended to additional cell line model systems. To study the effect of inhibiting endogenous BRCA1 function on psoriasin expression, the BG1-neo ovarian cell line that constitutively expresses a BRCA1 antisense construct (AS4 cells) and an isogenic vector transfected control cell line (BG1-neo cells) was used and psoriasin expression analysed in each model. BRCA1 antisense transfected AS4 cell line demonstrated increased expression of psoriasin at a protein level when compared to the vector only control cell line (Figure 2d). To confirm that BRCA1 was required to suppress psoriasin in a third model system, a BRCA1-specific siRNA oligonucleotide was used to abrogate protein expression in the T47D breast cancer cell line. In agreement with the BG1 cell line model, reduction of BRCA1 protein expression in the T47D cell line resulted in upregulation of psoriasin expression (figure 2e). Finally, it was investigated whether a BRCA1 disease-associated mutation would affect its ability to repress psoriasin expression. BRCA1-mutant HCC1937 were stably transfected cells with full length exogenous BRCA1 encoding a disease associated mutation, E1748A and its effect evaluated on psoriasin expression. Mutant BRCA1 completely failed to repress psoriasin suggesting that psoriasin represents a physiologically important BRCA1 target (Figure 2f).

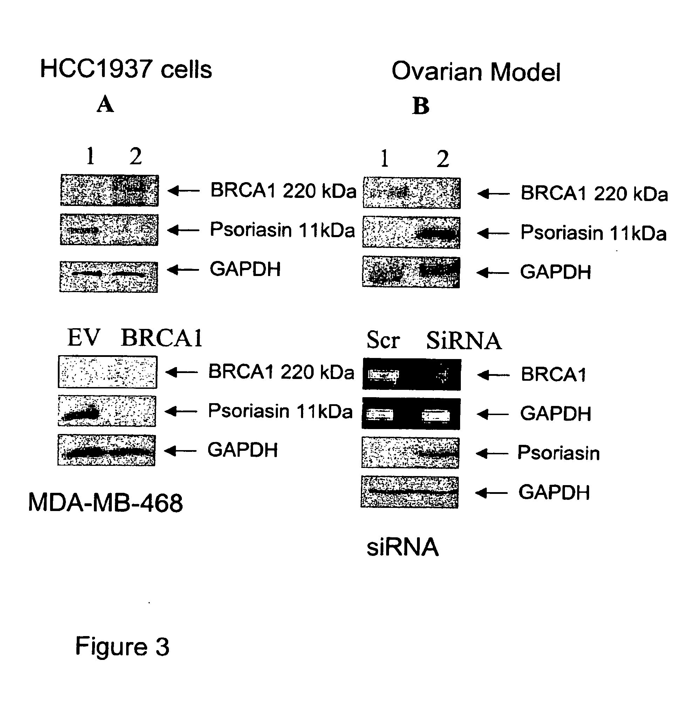

In figures 3A and 3B it is shown in multiple model systems that where BRCA1 is present expression of S100A7 (Psoriasin) is repressed.

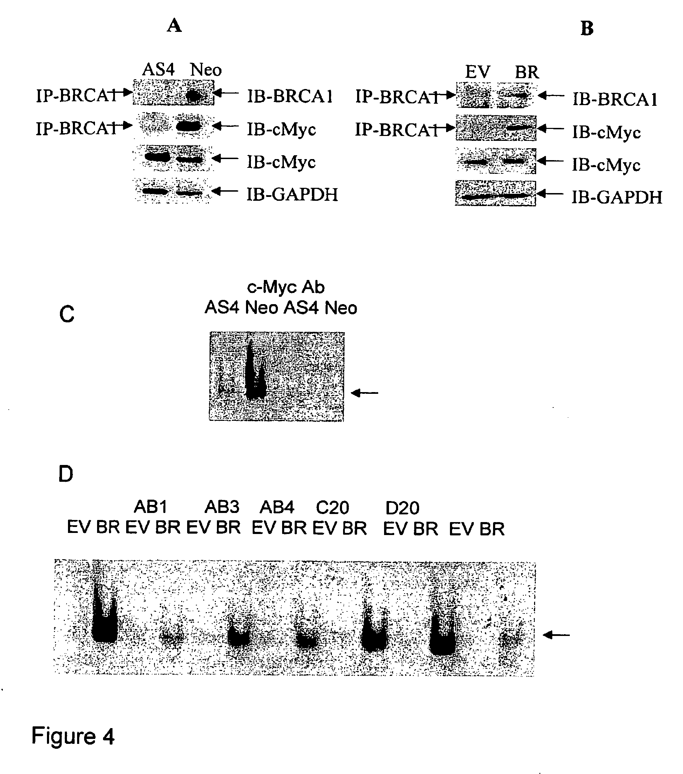

As shown in Figure 4 co-immunoprecipitation Western blots confirm an enhanced interaction between BRCA1-and c-Myc and demonstrate that BRCA1 forms a repressor complex with C-Myc on the psoriasin promoter.Reprobing for GAPDH on whole cell lysate confirmed equal protein loading.

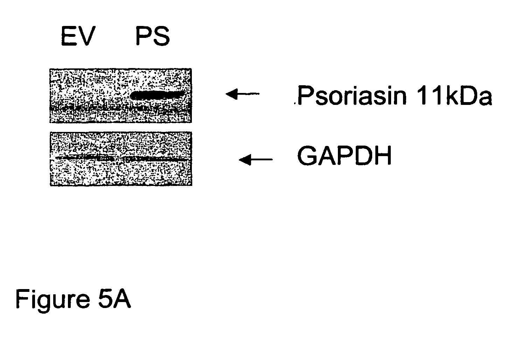

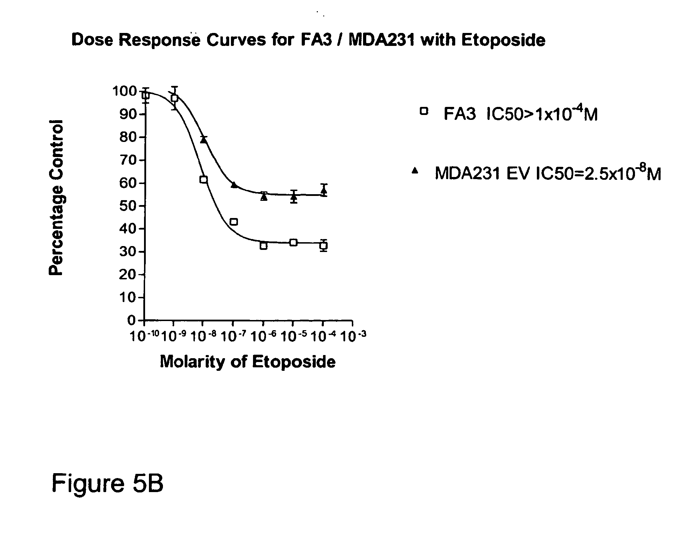

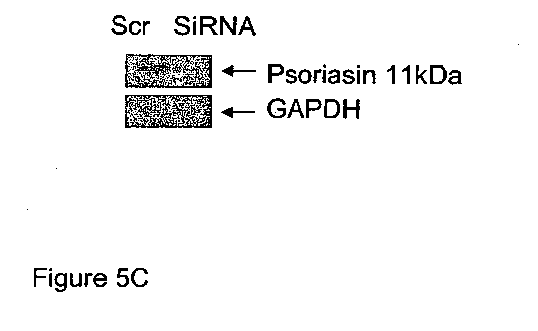

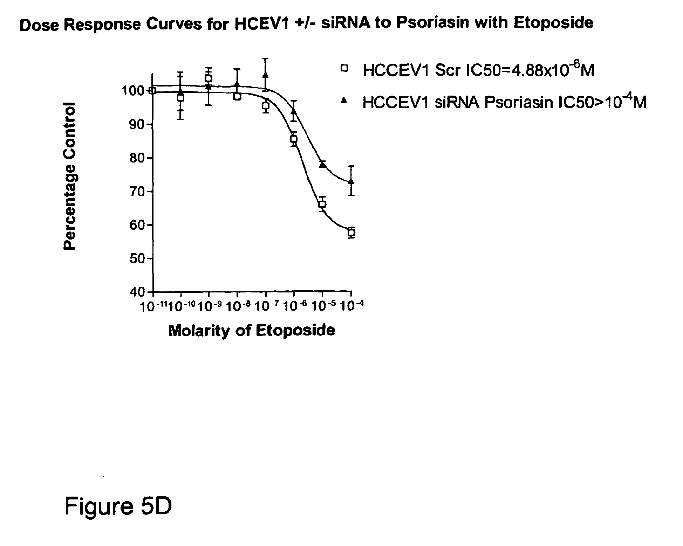

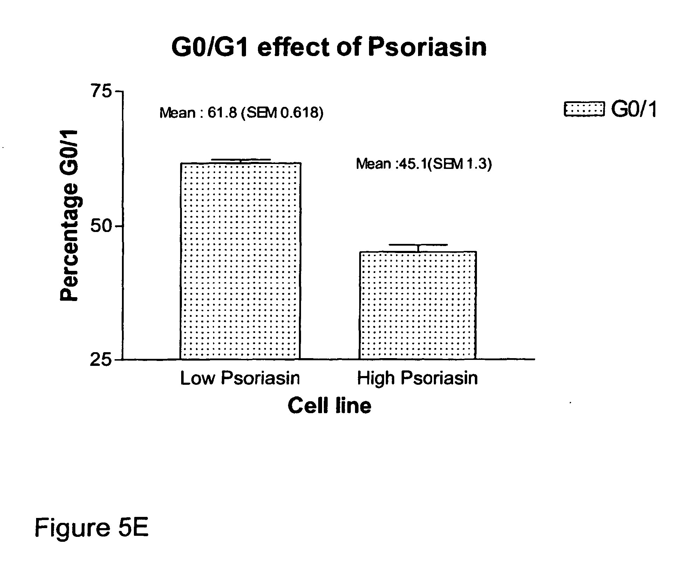

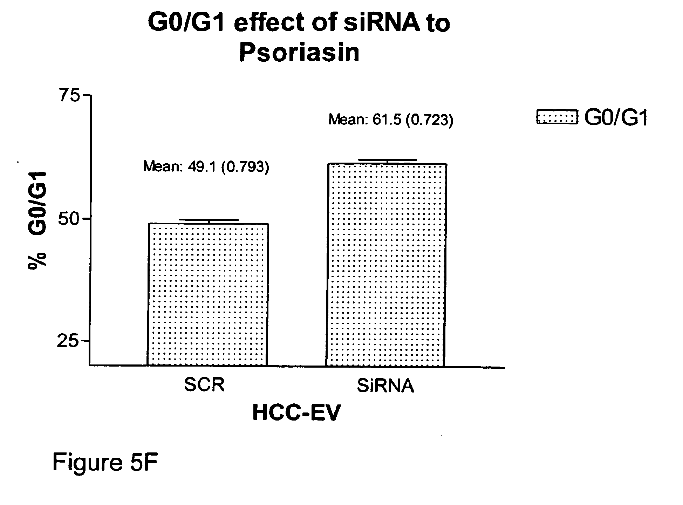

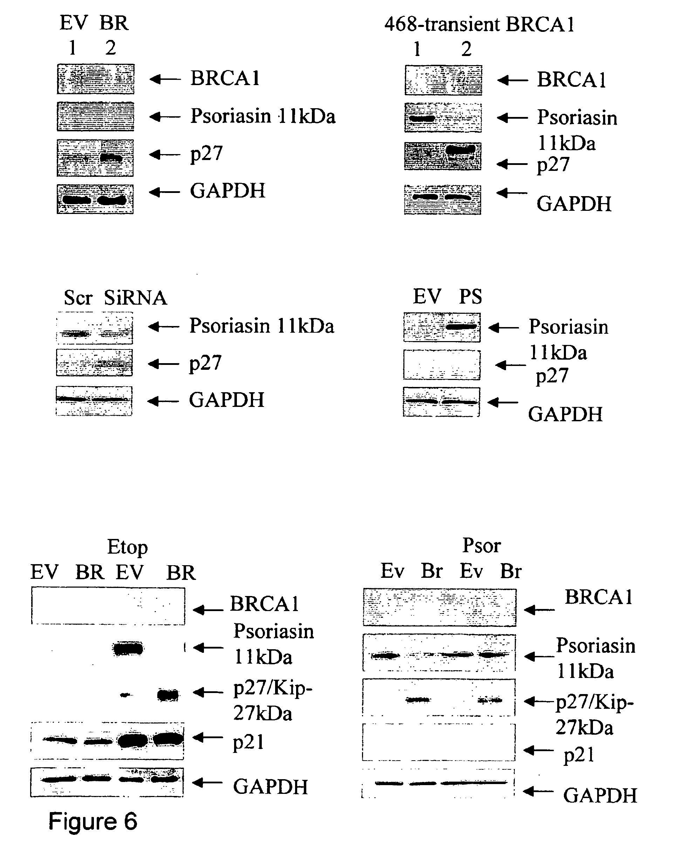

In figure 5 and figure 6 the expression profile of *psoriasin in response to etoposide is illustrated* indicating that psoriasin overexpression mediates sensitivity to etoposide and conversely the inhibition of endogenous psoriasin results in resistance to etoposide. Further. *psoriasin* modulates sensitivity/resistance to etoposide through the modulation of p27 expression levels.

### Psoriasin Mediates Sensitivity to Etoposide

The microarray data also indicated that psoriasin was etoposide inducible in the absence of functional BRCA1. As BRCA1-deficient cells have been reported to be highly sensitive to etoposide, in order to explore whether psoriasin plays a role in mediating the cellular response to etoposide, the expression *of psoriasin mRNA and protein following* etoposide treatment of BRCA1-deficient and reconstituted cells was examined. Northern and Western blot analysis confirmed enhanced expression of psoriasin mRNA and protein in BRCA1 deficient cells following etoposide treatment (Figure 7a & 7b).

To examine if psoriasin expression (as observed in BRCA1 deficient cells) affected sensitivity to etoposide, dose response assays in MDA-MB231 cells stably expressing exogenous psoriasin (HP1) compared to vector only transfected controls (MDA-MB231-zeo) were carried out. *Expression of psoriasin induced a* 4000-fold increase in sensitivity to etoposide (figure 7c) suggesting that it represents a functionally important BRCA1 target. Consistent with this observation inhibition of endogenous psoriasin in the BRCA1 deficient, etoposide sensitive HCC-EV cells induced a greater than 20-fold increase in resistance to etoposide (Figure 7d). Collectively these data suggest that loss of BRCA1-mediated repression of psoriasin results in enhanced sensitivity to etoposide implicating psoriasin as a functionally important BRCA1 target.

### MATERIALS AND METHODS:

### Generation of cell lines

HCC-Empty Vector and HCC-BRCA1 cells were generated by the stable transfection of the HCC1937 breast cancer cell line with the Rc/CMV (Invitrogen) or Rc/CMV-BRCA1 constructs under selection with geneticin (G418) (Sigma). The Rc/CMV construct that expresses the neomycin resistance gene was obtained from Invitrogen and the Rc/CMV-BRCA1 construct was generated as previously described (17). These constructs were transfected into the HCC1937 cell line using Genejuice methodology (Invitrogen) according to manufacturer's instructions. Transfected cells were selected in 0.2mg/ml G418, assessed by RT-PCR for expression of BRCA1 and the resultant HCC-EV and HCC-BR were selected.

### Cell Culture

The HCC1937 and T47D breast cancer cell lines were maintained in RPMI supplemented with 20% Foetal Calf Serum, 1mM sodium pyruvate and 100µg/ml penicillin-streptomycin (all from Life Technologies, Inc). The HCC-EV1, EV2, EV3, BR116, BR18 and BR-mixed cell lines were grown in HCC1937 medium supplemented with 0.2mg/ml G418.

### Flow Cytometry Analysis

DNA Content was evaluated following propidium iodide staining of cells as previously described. In all cases FACS analysis was carried out using an EPICS ELITE flow cytometer (Coulter).

### Drug sensitivity and growth assays

For drug sensitivity assays HCC-EV and HCCBR cells were seeded onto 24-well tissue culture plates at a density of 100,000 cells per well. After 24 hours, cells were incubated in HCC1937 medium supplemented with the described concentration ranges of etoposide (Bristol Myers Squibb). After 72 hours of continuous drug exposure, cells were detached with trypsin and counted using a Z2 particle and size analyser (Coulter, Miami, FL). IC₃₅₋₅₀ values were calculated for each drug from the respective sigmoidal dose response curves using Prism software.

### Antibodies and Western blot analysis

HCC-EV and HCC-BR cells were treated in the presence or absence of 1µM etoposide. Protein lysates were extracted in RIPA buffer (50µM hepes, pH 7.0, 150mM NaCl, 0.1% triton X-100, 0.1% sodium deoxycholate, 0.1% SDS) separated on a 12% SDS polyacrylamide gel and transferred to PVDF membrane followed by immunoblotting. BRCA1 westerns were carried out using the rabbit polyclonal, BRCA1 N-Terminal antibody, D-20 (Santa Cruz). BRCA1 IP Westerns were carried out using the rabbit polyclonal C-20 (Santa Cruz) and the mouse monoclonal AB1 (Calbiochem). Psoriasin Westerns were carried out using Imagenex antibody IMG-409A (Clone 47C1068). S100A8 Westerns were carried out using the Bachem antibody MRP8 (Clone 8-5C2) and S100A9 Westerns were carried out using the Bachem antibody MRP14 (Clone S36.48).

### SiRNA

T47D cells were transfected with a BRCA1 specific siRNA oligonucleotide or a control oligonucleotide and treated with a range of paclitaxel concentrations. After 72 hours of continuous drug exposure, cells were detached using trypsin and counted using a Z2 particle and size analyser (Coulter, Miami, FL). IC₃₀₋₅₀ values were calculated from the respective sigmoidal dose response curves. BRCA1 siRNA oligonucleotide 5'- aac ctg tct cca caa agt gtg - 3' Control siRNA oligonucleotide 5'- aaa acc cgu cua ggc ugu uac - 3'

### Etoposide Sensitivity Studies

The HCC-EV1 cell line was treated with scrambled or psoriasin specific siRNA and after 24 hours seeded at 100,000 cells per well on 24 well tissue culture plates. The MDA-MB-231-zeo and HP1 cell lines cells were seeded onto 24-well tissue culture plates at a density of 100,000 cells/well. Cells were then incubated in regular medium supplemented with etoposide in a concentration range from 100pM to 100µm. After 72 h of continuous drug exposure, cells were detached with trypsin, and counted using a Z2 particle and size analyzer (Coulter, Miami, FL). Each dose inhibition study was repeated three times. IC50 values were calculated from the respective sigmoidal dose-response curves using Prism software.

### Microarray analysis

Total RNA (10µg) derived from the HCC-BR116 and HCC-EV1 cells treated with either with etoposide 1µm for 24 hours or no chemotherapy was biotin labelled and fragmented according to standard protocols. Labelled cRNA was hybridized to Affymetrix Human Genome U133 Plus 2.0 arrays. The hybridized arrays were scanned using a GeneChip Scanner 3000. The data was converted to expression measures using the MAS5.0 algorithim (GeneChip operating software version 1.1) and raw data imported into the GeneSpring data analysis program version 6.1 (Silicon Genetics). Normalization was carried out to remove variability in the data, by dividing each measurement by the 50^{th} percentile of all measurements in that sample and then by dividing each gene by the median of its measurements in all samples. Following normalization the data was log transformed using the natural log. Genes were considered for further analysis based on standard GeneSpring filtering methods. Genes isolated for further analysis were required to have acceptable Affymetrix flag calls (present or marginal in all samples), greater than 2-fold differential expression compared to control (in at least one sample), a t-test p value below 0.05 (in all samples) and that they passed an error filter based on the Rocke-Lorenzato model (in all samples). The list of genes that satisfied the above specifications and the samples, were clustered using a hierarchical clustering algorithim utilising Pearson Correlation as the similarity measure, giving rise to a 2-dimensional dendrogram.

The invention is not limited to the embodiments hereinbefore described which may be varied construction, detail and process step.

## Claims

1. A method of predicting the presence of a non-functional BRCA1 gene in a test biological sample comprising the steps of:
assaying the test sample to determine an expression profile of at least one member of the S100 family of genes selected from the group comprising S100A7, S100A8, S100A9, S100A10 or S100A2, and
comparing the expression profile of at least one member with the expression profile of said member in a control biological sample,
wherein an increase in the expression of any one of S100A7, S100A8, S1009A9 or S100A10 or a decrease in expression of S100A2 in the test sample being assayed is indicative that BRCA1 is non-functional.

2. The method as claimed in Claim 1 in which the biological sample is selected from the group comprising: tissue; whole blood; and serum.

3. The method as claimed in any one of Claims 1 or 2 in which the assaying step comprises assaying for mRNA transcripts of the gene of at least one member of the S100 family of proteins selected from the group comprising S100A7, S100A8, S100A9, S100A10 or S100A2 using RT-PCR.

4. The method as claimed in any one of the preceding claims in which the assaying step comprises probing the sample with an antibody to at least one member of the S100 family of proteins selected from the group comprising S100A7, S100A8, S100A9, S100A10 or S100A2.

5. The method as claimed in Claim 4 in which expression of the protein is assayed using an ELISA.

6. The method as claimed in any preceding claim wherein the expression profiles of at least two members of the S100 family selected from the group of proteins comprising S100A7, S100A8, S100A9, S100A10 or S100A2 are determined.

## Patentansprüche

1. Ein Verfahren zum Vorhersagen des Vorhandenseins eines nichtfunktionellen BRCA1-Gens in einer biologischen Untersuchungsprobe, das die folgenden Schritte beinhaltet:
Prüfen der Untersuchungsprobe, um ein Expressionsprofil von mindestens einem Mitglied der S100-Familie von Genen, ausgewählt aus der Gruppe, die S100A7, S100A8, S100A9, S100A10 oder S100A2 beinhaltet, zu bestimmen, und
Vergleichen des Expressionsprofils von mindestens einem Mitglied mit dem Expressionsprofil des Mitglieds in einer biologischen Stichprobe,
wobei eine Erhöhung der Expression von einem aus S100A7, S100A8, S100A9 oder S100A10 oder eine Verringerung der Expression von S100A2 in der geprüften Untersuchungsprobe anzeigt, dass BRCA1 nichtfunktionell ist.

2. Verfahren gemäß Anspruch 1, wobei die biologische Probe aus der Gruppe ausgewählt wird, die Folgendes beinhaltet: Gewebe, Vollblut und Serum.

3. Verfahren gemäß einem der Ansprüche 1 oder 2, wobei der Prüfschritt das Prüfen auf mRNA-Abschriften der Gene von mindestens einem Mitglied der S100-Familie von Proteinen, ausgewählt aus der Gruppe, die S100A7, S100A8, S100A9, S100A10 oder S100A2 beinhaltet, unter Verwendung von RT-PCR beinhaltet.

4. Verfahren gemäß einem der vorhergehenden Ansprüche, wobei der Prüfschritt das Untersuchen der Probe mit einem Antikörper auf mindestens ein Mitglied der S100-Familie von Proteinen, ausgewählt aus der Gruppe, die S100A7, S100A8, S100A9, S100A10 oder S100A2 beinhaltet, beinhaltet.

5. Verfahren gemäß Anspruch 4, wobei die Expression des Proteins unter Verwendung von ELISA geprüft wird.

6. Verfahren gemäß einem der vorhergehenden Ansprüche, wobei die Expressionsprofile von mindestens zwei Mitgliedern der S100-Familie, ausgewählt aus der Gruppe von Proteinen, die S100A7, S100A8, S100A9, S100A10 oder S100A2 beinhaltet, bestimmt werden.

## Revendications

1. Une méthode pour prédire la présence d'un gène BRCA1 non fonctionnel dans un échantillon biologique pour essai comprenant les étapes de :
analyser l'échantillon pour essai afin de déterminer un profil d'expression d'au moins un élément de la famille S100 de gènes sélectionné dans le groupe comprenant S100A7, S100A8, S100A9, S100A10 ou S100A2, et
comparer le profil d'expression d'au moins un élément avec le profil d'expression dudit élément dans un échantillon biologique témoin,
où une augmentation de l'expression de n'importe quel élément parmi S100A7, S100A8, S100A9 ou S100A10 ou une diminution de l'expression de S100A2 dans l'échantillon pour essai en cours d'analyse sont indicatives que BRCA1 est non fonctionnel.

2. La méthode telle que revendiquée dans la revendication 1 dans laquelle l'échantillon biologique est sélectionné dans le groupe comprenant : tissu ; sang entier ; et sérum.

3. La méthode telle que revendiquée dans n'importe laquelle des revendications 1 ou 2 dans laquelle l'étape d'analyse comprend détecter par analyse des transcrits d'ARNm du gène d'au moins un élément de la famille S100 de protéines sélectionné dans le groupe comprenant S100A7, S100A8, S100A9, S100A10 ou S100A2 à l'aide de la technique de RT-PCR.

4. La méthode telle que revendiquée dans n'importe laquelle des revendications précédentes dans laquelle l'étape d'analyse comprend sonder l'échantillon avec un anticorps contre au moins un élément de la famille S100 de protéines sélectionné dans le groupe comprenant S100A7, S100A8, S100A9, S100A10 ou S100A2.

5. La méthode telle que revendiquée dans la revendication 4 dans laquelle l'expression de la protéine est analysée à l'aide d'une technique ELISA.

6. La méthode telle que revendiquée dans n'importe quelle revendication précédente où les profils d'expression d'au moins deux éléments de la famille S100 sélectionnés dans le groupe de protéines comprenant S100A7, S100A8, S100A9, S100A10 ou S100A2 sont déterminés.
